# EUROPEAN PATENT SPECIFICATION

(11) **EP 3 894 064 B1**
(45) Date of publication and mention of the grant of the patent: **15.11.2023**
(21) Application number: 20725207.3
(22) Date of filing: 19.05.2020
(51) Int. Cl.: B01J 13/16, A01N 25/28, A61K 8/11, A61K 9/50, C11D 3/50

(54) **PROCESS FOR PREPARING MICROCAPSULES**
VERFAHREN ZUR HERSTELLUNG VON MIKROKAPSELN
PROCÉDÉ DE PRÉPARATION DE MICROCAPSULES

(30) Priority: 21.05.2019 US 201962850654 P; 05.07.2019 EP 19184788
(43) Date of publication of application: 20.10.2021
(73) Proprietor: Firmenich SA, 1242 Satigny (CH)
(72) Inventor: MA, Ling, Cranbury, NJ 08512 (US); FENG, Jingyu, Plainsboro, NJ 08536 (US); OUALI, Lahoussine, 1242 Satigny (CH); JERRI, Huda, Plainsboro, New Jersey 08536 (US)
(74) Representative: Dureisseix, Valérie
(86) International application number: PCT/EP2020/063888
(87) International publication number: WO 2020/234262

(56) References cited:
- WO-A1-2018/123349
- US-A- 5 304 448
- US-A1- 2018 343 855

## Description

### Technical Field

The present invention relates to a new process for the preparation of a microcapsule slurry. Perfuming compositions and consumer products comprising microcapsules, in particular perfumed consumer products in the form of home care or personal care products, are also part of the invention.

### Background of the Invention

One of the problems faced by the perfumery industry lies in the relatively rapid loss of olfactive benefit provided by odoriferous compounds due to their volatility, particularly that of "top-notes". In order to tailor the release rates of volatiles, delivery systems such as microcapsules containing a perfume are needed to protect and later release the core payload when triggered. A key requirement from the industry regarding these systems is to survive suspension in challenging bases without physically dissociating or degrading. This is referred to as *stability* for the delivery system. For instance, fragranced personal and household cleansers containing high levels of aggressive surfactant detergents are very challenging for the stability of microcapsules.

Polyurea and polyurethane-based microcapsule slurry are widely used for example in perfumery industry as they provide a long lasting pleasant olfactory effect after their applications on different substrates. Those microcapsules have been widely disclosed in the prior art (see for example WO2007/004166 or EP 2300146 from the Applicant). WO2018/123349A1, US2018/343855A1 and WO2012/107323A1 disclose core-shell microcapsules comprising a hydrophobic ingredient, and processes to produce them.

There is still a need to provide new microcapsules, while not compromising on the performance of the microcapsules, in particular in terms of stability in a challenging medium such as a consumer product base, as well as in delivering a good performance in terms of active ingredient delivery, e.g. olfactive performance in the case of perfuming ingredients.

The present invention is proposing a solution by providing new microcapsules and a process for preparing said microcapsules.

The process of the invention allows preparing microcapsules having an oil-based core and a shell comprising a polymerized amino-acid derived polyisocyanate having at least two isocyanate functional groups, said microcapsules having the desired stability in challenging bases.

A first object of the invention relates therefore to a process for the preparation of a core-shell microcapsule slurry comprising the following steps:
a) dissolving at least an amino-acid derived polyisocyanate having at least two isocyanate functional groups in a hydrophobic material, preferably a perfume to form an oil phase;
b) preparing a dispersing phase comprising a stabilizer, wherein the dispersing phase is not miscible with the oil phase;
c) adding the oil phase into the dispersing phase to form a two-phases dispersion;
d) optionally, adding a reactant to the dispersion obtained in step c); and
e) performing a curing step to form core-shell microcapsules in the form of a slurry.

Disclosed but not claimed is a core-shell microcapsule slurry obtainable by the process as defined above.

A second object of the invention is a core-shell microcapsule comprising:
- an oil-based core comprising a hydrophobic material, preferably comprising a perfume oil, and
- a shell comprising at least a polymerized amino-acid derived polyisocyanate having at least two isocyanate functional groups:wherein the amino-acid derived polyisocyanate is lysine diisocyanate or chosen in the group consisting of arginine triisocyanate, asparagine triisocyanate, proline 5 triisocyanate, glutamine triisocyanate, lysine triisocyanate, lysine ethyl ester triisocyanate, lysine methyl ester triisocyanate, lysine propyl ester triisocyanate, or derivatives thereof, and mixtures thereof.

Disclosed but not claimed is a core-shell microcapsule slurry comprising at least one microcapsule made of:
- an oil-based core comprising a hydrophobic material, preferably comprising a perfume oil, and
- a shell comprising at least a polymerized amino-acid derived polyisocyanate having at least two isocyanate functional groups.

A third fifthobj ect of the invention is a perfuming composition comprising the microcapsule as defined above, wherein the core comprises a perfume.

Another object of the invention is a consumer product comprising the microcapsule or a perfuming composition as defined above.

### Drawings

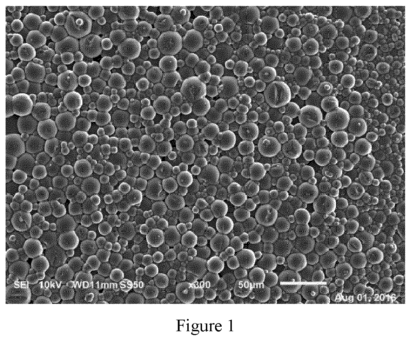

**Figure 1****:** Microscopic picture of microcapsules according to the invention.

### Detailed description of the invention

Unless stated otherwise, percentages (%) are meant to designate a percentage by weight of a composition.

By "**hydrophobic material**", it is meant any hydrophobic material - single material or a mixture of material - which forms a two-phase dispersion when mixed with water.

By "**ingredient**", it is meant a single compound or a combination of ingredients.

By "**perfume or flavour oil**", it is meant a single perfuming or flavouring compound or a mixture of several perfuming or flavouring compounds.

By "**consumer product**" or "**end-product**" it is meant a manufactured product ready to be distributed, sold and used by a consumer.

For the sake of clarity, by the expression "dispersion" in the present invention it is meant a system in which particles are dispersed in a continuous phase of a different composition and it specifically includes a suspension or an emulsion.

A "**microcapsule**", or the similar, in the present invention it is meant that core-shell microcapsules have a particle size distribution in the micron range (e.g. a mean diameter(D [4,3]) comprised between about 1 and 3000 microns) and comprise an external solid polymer-based shell and an internal continuous oil phase enclosed by the external shell.

By "**an amino-acid derived polyisocyanate**", it is meant a polyisocyanate prepared/synthesized/obtained from an amino-acid.

By "**polyurea-based**" wall or shell, it is meant that the polymeric shell comprises urea linkages, urea linkages being produced by amino groups capable of further reacting with isocyanate groups during interfacial polymerization.

By "**polyurethane-based**" wall or shell, it is meant that the polymeric shell contains urethane linkages produced by reaction with polyols.

By "**polyaminoester**" or "**polyaminoester cross-linker**" which are used indifferently, it is meant a compound having preferably the following formula: 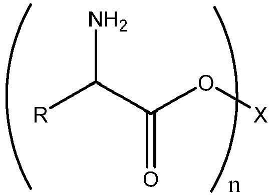
with n≥2, wherein R represents a hydrogen atom, a benzyl, a methyl, an iso-propyl, an iso-butyl or a sec-butyl group;
and wherein X is derivatived from a polyol of formula (HO)ₙ-X.

Preferably X is a C₁-C₁₀ hydrocarbon optionally substituted with 1 to 10 hydroxy groups or a - ((CH₂)ₚ-O)ₘ-(CH₂)ₚ group wherein p is an integer between 1 and 10 and m is an integer between 1 and 100. Preferably, X may be a C₁-C₁₀ alkane-di/tri/tetra/penta/hexa-yl group or a -((CH₂)ₚ-O)ₘ-(CH₂)ₚ group wherein p may be an integer between 1 and 5 and m may be an integer between 1 and 50. Even more preferably, X may be a C₁-C₅ alkane-di/tri/tetra-yl group or a - ((CH₂) ₚ-O)ₘ-(CH₂)ₚ group wherein p may be an integer between 1 and 3 and m may be an integer between 1 and 10. Even more preferably, X may be a C₁-C₅ alkane-di/tri/tetra-yl group or a -((CH₂) ₚ-O)ₘ-(CH₂)ₚ group wherein p may be an 2 and m may be an integer between 1 and 5.

According to any one embodiment, n is an integer between 2 and 10. Preferably, n is an integer between 1 and 4.

The polyol has preferably the following formula: HO-((CH₂) ₚ-O)ₘ-(CH₂)ₚ-OH with n = 2 and with p and m being as defined above.

The polyaminoester is preferably obtained by the reaction between a polyol and an amino-acid. As non-limiting examples, the polyol can be chosen in the group consisting of glycerol, pentaerythritol, 1,1,1-tris(hydroxymethyl)ethane, 1,4-butanediol, diethylene glycol, and mixtures thereof.

As non-limiting examples, the amino-acid can be chosen in the group consisting of glycine, phenylalanine, alanine, valine, leucine, isoleucine and mixtures thereof.

According to a particular embodiment, the polyaminoester is chosen in the group consisting of the following compounds:

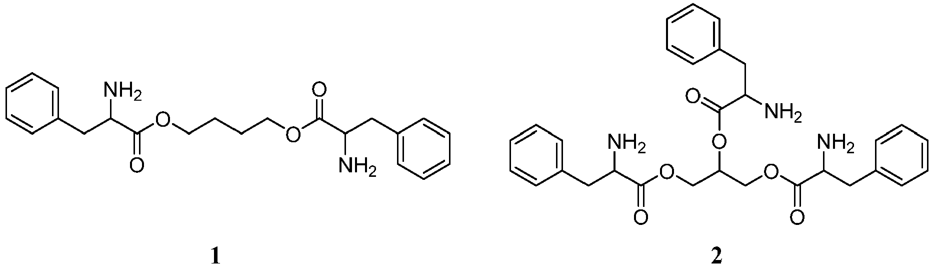

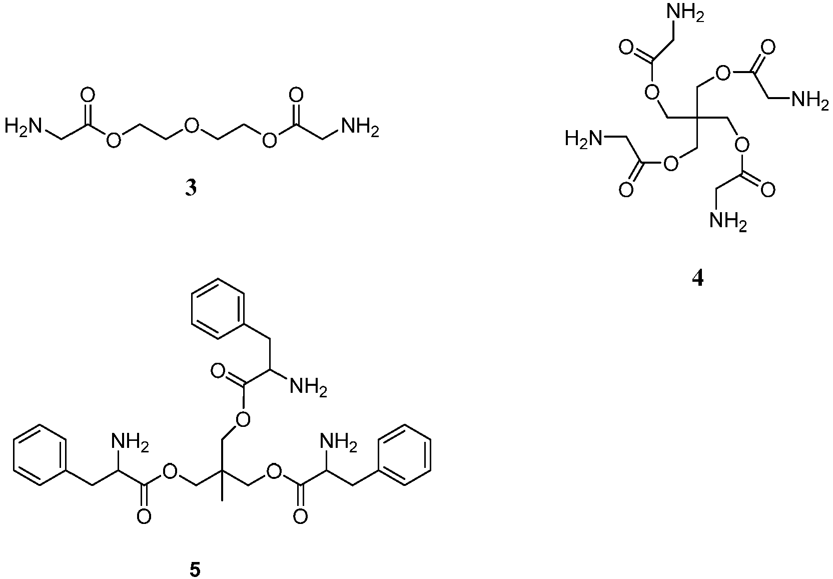

Thus, according to this embodiment, the polyaminoester is chosen in the group consisting of butane-1,4-diyl bis(2-amino-3-phenylpropanoate (**compound 1**), propane-1,2,3-triyl tris(2-amino-3-phenylpropanoate) (**compound 2**), oxybis(ethane-2,1-diyl) bis(2-aminoacetate) (**compound 3**), 2,2 bis((glycyloxy)methyl)propane-1,3-diyl bis(2-aminoacetate) (**compound 4**), [3-(2-amino-3-phenyl-propanoyl)oxy-2-[(2-amino-3-phenyl-propanoyl)oxymethyl]-2-methylpropyl] 2-amino-3-phenyl-propanoate (**compound 5**) and mixtures thereof.

Methods for preparing such compounds are well-known in the literature. One may cite for example, *Phenylalanine-Based Poly (ester urea): Synthesis, Characterization, and in vitro Degradation,* Jiayi Yu, Fei Lin, Panpan Lin, Yaohua Gao, and Matthew L. Becker, Macromolecules 2014, 47, 121-129 to prepare compound 1.

The synthesis of compound 1 can also be found in the literature: *Versatile biodegradable poly (ester amide)s derived from alpha-amino acids for vascular tissue engineering,* Karimi P, Rizkalla AS, Mequanint K., Materials 2010;3:2346-68*.*

Synthesis of compound 3 can be found in *Novel Poly(ester amide)s From Glycine and L-lactic acid by an Easy and Cost-effective synthesis,* Ana C. Fonseca et al. Polym Int 2013; 62:736-743 *Page 23.*

### Process for preparing a microcapsule slurry

A first object of the invention is therefore a process for the preparation of a core-shell microcapsule slurry comprising the following steps:
a) dissolving at least an amino-acid derived polyisocyanate having at least two isocyanate functional groups in a hydrophobic material, preferably a perfume to form an oil phase;
b) preparing a dispersing phase comprising a stabilizer, wherein the dispersing phase is not miscible with the oil phase;
c) adding the oil phase into the dispersing phase to form a two-phases dispersion;
d) optionally, adding a reactant to the dispersion obtained in step c); and
e) performing a curing step to form core-shell microcapsules in the form of a slurry.

In one step of the process, at least an amino-acid derived polyisocyanate having at least two isocyanate functional groups is dissolved in a hydrophobic material, preferably a perfume, to form an oil phase.

### Hydrophobic material

Hydrophobic material according to the invention can be "inert" material like solvents or active ingredients.

When, hydrophobic materials are active ingredient, it is preferably chosen from the group consisting of flavor, flavor ingredients, perfume, perfume ingredients, nutraceuticals, cosmetics, pest control agents, biocide actives and mixtures thereof.

According to a particular embodiment, the hydrophobic material comprises a mixture of a perfume with another ingredient selected from the group consisting of nutraceuticals, cosmetics, pest control agents and biocide actives.

According to a particular embodiment, the hydrophobic material comprises a mixture of biocide actives with another ingredient selected from the group consisting of perfume, nutraceuticals, cosmetics, pest control agents.

According to a particular embodiment, the hydrophobic material comprises a mixture of pest control agents with another ingredient selected from the group consisting of perfume, nutraceuticals, cosmetics, biocide actives.

According to a particular embodiment, the hydrophobic material comprises a perfume.

According to a particular embodiment, the hydrophobic material consists of a perfume.

According to a particular embodiment, the hydrophobic material consists of biocide actives.

According to a particular embodiment, the hydrophobic material consists of pest control agents.

By "perfume" (or also "perfume oil") what is meant here is an ingredient or composition that is a liquid at about 20°C. According to any one of the above embodiments said perfume oil can be a perfuming ingredient alone or a mixture of ingredients in the form of a perfuming composition. As a "perfuming ingredient" it is meant here a compound, which is used for the primary purpose of conferring or modulating an odour. In other words such an ingredient, to be considered as being a perfuming one, must be recognized by a person skilled in the art as being able to at least impart or modify in a positive or pleasant way the odor of a composition, and not just as having an odor. For the purpose of the present invention, perfume oil also includes combination of perfuming ingredients with substances which together improve, enhance or modify the delivery of the perfuming ingredients, such as perfume precursors, emulsions or dispersions, as well as combinations which impart an additional benefit beyond that of modifying or imparting an odor, such as long-lasting, blooming, malodour counteraction, antimicrobial effect, microbial stability, pest control.

The nature and type of the perfuming ingredients present in the oil phase do not warrant a more detailed description here, which in any case would not be exhaustive, the skilled person being able to select them on the basis of its general knowledge and according to intended use or application and the desired organoleptic effect. In general terms, these perfuming ingredients belong to chemical classes as varied as alcohols, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said ingredients may also be compounds known to release in a controlled manner various types of perfuming compounds.

In particular one may cite perfuming ingredients which are commonly used in perfume formulations, such as:
- Aldehydic ingredients: decanal, dodecanal, 2-methyl-undecanal, 10-undecenal, octanal, nonanal and/or nonenal;
- Aromatic-herbal ingredients: eucalyptus oil, camphor, eucalyptol, 5-methyltricyclo[6.2.1.0-2,7-]undecan-4-one, 1-methoxy-3-hexanethiol, 2-ethyl-4,4-dimethyl-1,3-oxathiane, 2,2,7/8,9/10-Tetramethylspiro[5.5]undec-8-en-1-one, menthol and/or alpha-pinene;
- Balsamic ingredients: coumarin, ethylvanillin and/or vanillin;
- Citrus ingredients: dihydromyrcenol, citral, orange oil, linalyl acetate, citronellyl nitrile, orange terpenes, limonene, 1-p-menthen-8-yl acetate and/or 1,4(8)-p-menthadiene;
- Floral ingredients: methyl dihydrojasmonate, linalool, citronellol, phenylethanol, 3-(4-tert-butylphenyl)-2-methylpropanal, hexylcinnamic aldehyde, benzyl acetate, benzyl salicylate, tetrahydro-2-isobutyl-4-methyl-4(2H)-pyranol, beta ionone, methyl 2-(methylamino)benzoate, (E)-3-methyl-4-(2,6,6-trimethyl-2-cyclohexen-1-yl)-3-buten-2-one, (1E)-1-(2,6,6-trimethyl-2-cyclohexen-1-yl)-1-penten-3-one, 1-(2,6,6-trimethyl-1,3-cyclohexadien-1-yl)-2-buten-1-one, (2E)-1-(2,6,6-trimethyl-2-cyclohexen-1-yl)-2-buten-1-one, (2E)-1-[2,6,6-trimethyl-3-cyclohexen-1-yl]-2-buten-1-one, (2E)-1-(2,6,6-trimethyl-1-cyclohexen-1-yl)-2-buten-1-one, 2,5-dimethyl-2-indanmethanol, 2,6,6-trimethyl-3-cyclohexene-1-carboxylate, 3-(4,4-dimethyl-1-cyclohexen-1-yl)propanal, hexyl salicylate, 3,7-dimethyl-1,6-nonadien-3-ol, 3-(4-isopropylphenyl)-2-methylpropanal, verdyl acetate, geraniol, p-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate , high cis methyl dihydrojasmonate, 3-methyl-5-phenyl-1-pentanol, verdyl proprionate, geranyl acetate, tetrahydro linalool, cis-7-p-menthanol, propyl (S)-2-(1,1-dimethylpropoxy)propanoate, 2-methoxynaphthalene, 2,2,2-trichloro-1-phenylethyl acetate, 4/3-(4-hydroxy-4-methylpentyl)-3-cyclohexene-1-carbaldehyde, amylcinnamic aldehyde, 8-decen-5-olide, 4-phenyl-2-butanone, isononyle acetate, 4-(1,1-dimethylethyl)-1-cyclohexyl acetate, verdyl isobutyrate and/or mixture of methylionones isomers;
- Fruity ingredients: gamma-undecalactone, 2,2,5-trimethyl-5-pentylcyclopentanone, 2-methyl-4-propyl-1,3-oxathiane, 4-decanolide, ethyl 2-methyl-pentanoate, hexyl acetate, ethyl 2-methylbutanoate, gamma-nonalactone, allyl heptanoate, 2-phenoxyethyl isobutyrate, ethyl 2-methyl-1,3-dioxolane-2-acetate, 3-(3,3/1,1-dimethyl-5-indanyl)propanal, diethyl 1,4-cyclohexanedicarboxylate, 3-methyl-2-hexen-1-yl acetate, 1-[3,3-dimethylcyclohexyl]ethyl [3-ethyl-2-oxiranyl]acetate and/or diethyl 1,4-cyclohexane dicarboxylate;
- Green ingredients: 2-methyl-3-hexanone (E)-oxime, 2,4-dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1-(5,5-dimethyl-1-cyclohexen-1-yl)-4-penten-1-one;
- Musk ingredients: 1,4-dioxa-5,17-cycloheptadecanedione, (Z)-4-cyclopentadecen-1-one, 3-methylcyclopentadecanone, 1-oxa-12-cyclohexadecen-2-one, 1-oxa-13-cyclohexadecen-2-one, (9Z)-9-cycloheptadecen-1-one, 2-{1S)-1-[(1R)-3,3-dimethylcyclohexyl]ethoxy}-2-oxoethyl propionate 3-methyl-5-cyclopentadecen-1-one, 1,3,4,6,7,8-hexahydro-4,6,6,7,8,8-hexamethyl-cyclopenta-g-2-benzopyrane, (1S,1'R)-2-[1-(3',3'-dimethyl-1'-cyclohexyl)ethoxy]-2-methylpropyl propanoate, oxacyclohexadecan-2-oneand/or (1S,1'R)-[1-(3',3'-dimethyl-1'-cyclohexyl)ethoxycarbonyl]methyl propanoate, ;
- Woody ingredients: 1-[(1RS,6SR)-2,2,6-trimethylcyclohexyl]-3-hexanol, 3,3-dimethyl-5-[(1R)-2,2,3-trimethyl-3-cyclopenten-1-yl]-4-penten-2-ol, 3,4'-dimethylspiro[oxirane-2,9'-tricyclo[6.2.1.0^{2,7}]undec[4]ene, (1-ethoxyethoxy)cyclododecane, 2,2,9,11-tetramethylspiro[5.5]undec-8-en-1-yl acetate, 1-(octahydro-2,3,8,8-tetramethyl-2-naphtalenyl)-1-ethanone, patchouli oil, terpenes fractions of patchouli oil, clearwood^{®}, (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, methyl cedryl ketone, 5-(2,2,3-trimethyl-3-cyclopentenyl)-3-methylpentan-2-ol, 1-(2,3,8,8-tetramethyl-1,2,3,4, 6,7,8,8a-octahydronaphthalen-2-yl)ethan-1-one and/or isobornyl acetate;
- Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal, 7-methyl-2H-1,5-benzodioxepin-3(4H)-one, 2,5,5-trimethyl-1,2,3,4,4a,5,6,7-octahydro-2-naphthalenol, 1-phenylvinyl acetate, 6-methyl-7-oxa-1-thia-4-azaspiro[4.4]nonan and/or 3-(3-isopropyl-1-phenyl)butanal.

It is also understood that perfuming ingredient may also be compounds known to release in a controlled manner various types of perfuming compounds also known as properfume or profragrance. Non-limiting examples of suitable properfume may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, trans-3-(dodecylthio)-1-(2,6,6-trimethyl-3-cyclohexen-1-yl)-1-butanone, 2-phenylethyl oxo(phenyl)acetate or a mixture thereof.

According to an embodiment, perfuming ingredients have a high steric hindrance and are chosen in particular from one of the following groups:
- Group 1: perfuming ingredients comprising a cyclohexane, cyclohexene, cyclohexanone or cyclohexenone ring substituted with at least one linear or branched C₁ to C₄ alkyl or alkenyl substituent;
- Group 2: perfuming ingredients comprising a cyclopentane, cyclopentene, cyclopentanone or cyclopentenone ring substituted with at least one linear or branched C₄ to C₈ alkyl or alkenyl substituent;
- Group 3: perfuming ingredients comprising a phenyl ring or perfuming ingredients comprising a cyclohexane, cyclohexene, cyclohexanone or cyclohexenone ring substituted with at least one linear or branched C₅ to C₈ alkyl or alkenyl substituent or with at least one phenyl substituent and optionally one or more linear or branched C₁ to C₃ alkyl or alkenyl substituents;
- Group 4: perfuming ingredients comprising at least two fused or linked C₅ and/or C₆ rings;
- Group 5: perfuming ingredients comprising a camphor-like ring structure;
- Group 6: perfuming ingredients comprising at least one C7 to C20 ring structure;
- Group 7: perfuming ingredients having a logP value above 3.5 and comprising at least one tert-butyl or at least one trichloromethyl substitutent;

Examples of ingredients from each of these groups are:
- Group 1: 2,4-dimethyl-3-cyclohexene-1-carbaldehyde (origin: Firmenich SA, Geneva, Switzerland), isocyclocitral, menthone, isomenthone, Romascone^{®} (methyl 2,2-dimethyl-6-methylene-1-cyclohexanecarboxylate, origin: Firmenich SA, Geneva, Switzerland), nerone, terpineol, dihydroterpineol, terpenyl acetate, dihydroterpenyl acetate, dipentene, eucalyptol, hexylate, rose oxide, Perycorolle^{®} ((S)-1,8-p-menthadiene-7-ol, origin: Firmenich SA, Geneva, Switzerland), 1-p-menthene-4-ol, (1RS,3RS,4SR)-3-p-mentanyl acetate, (1R,2S,4R)-4,6,6-trimethyl-bicyclo[3,1,1]heptan-2-ol, Doremox^{®} (tetrahydro-4-methyl-2-phenyl-2H-pyran, origin: Firmenich SA, Geneva, Switzerland), cyclohexyl acetate, cyclanol acetate, Fructalate^{®} (1,4-cyclohexane diethyldicarboxylate, origin: Firmenich SA, Geneva, Switzerland), Koumalactone^{®} ((3ARS,6SR,7ASR)-perhydro-3,6-dimethyl-benzo[B]furan-2-one, origin: Firmenich SA, Geneva, Switzerland), Natactone^{®} ((6R)-perhydro-3,6-dimethyl-benzo[B]furan-2-one, origin: Firmenich SA, Geneva, Switzerland), 2,4,6-trimethyl-4-phenyl-1,3-dioxane, 2,4,6-trimethyl-3-cyclohexene-1-carbaldehyde;
- Group 2: (E)-3-methyl-5-(2,2,3-trimethyl-3-cyclopenten-1-yl)-4-penten-2-ol (origin: Givaudan SA, Vernier, Switzerland), (1'R,E)-2-ethyl-4-(2',2',3'-trimethyl-3'-cyclopenten-1'-yl)-2-buten-1-ol (origin: Firmenich SA, Geneva, Switzerland), Polysantol^{®} ((1'R,E)-3,3-dimethyl-5-(2',2',3'-trimethyl-3'-cyclopenten-1'-yl)-4-penten-2-ol, origin: Firmenich SA, Geneva, Switzerland), fleuramone, Hedione^{®} HC (methyl-cis-3-oxo-2-pentyl-1-cyclopentane acetate, origin: Firmenich SA, Geneva, Switzerland), Veloutone^{®} (2,2,5-Trimethyl-5-pentyl-1-cyclopentanone, origin: Firmenich SA, Geneva, Switzerland), Nirvanol^{®} (3,3-dimethyl-5-(2,2,3-trimethyl-3-cyclopenten-1-yl)-4-penten-2-ol, origin: Firmenich SA, Geneva, Switzerland), 3-methyl-5-(2,2,3-trimethyl-3-cyclopenten-1-yl)-2-pentanol (origin, Givaudan SA, Vernier, Switzerland);
- Group 3: damascones, Neobutenone^{®} (1-(5,5-dimethyl-1-cyclohexen-1-yl)-4-penten-1-one, origin: Firmenich SA, Geneva, Switzerland), nectalactone ((1'R)-2-[2-(4'-methyl-3'-cyclohexen-1'-yl)propyl]cyclopentanone), alpha-ionone, beta-ionone, damascenone, Dynascone^{®} (mixture of 1-(5,5-dimethyl-1-cyclohexen-1-yl)-4-penten-1-one and 1-(3,3-dimethyl-1-cyclohexen-1-yl)-4-penten-1-one, origin: Firmenich SA, Geneva, Switzerland), Dorinone^{®} beta (1-(2,6,6-trimethyl-1-cyclohexen-1-yl)-2-buten-1-one, origin: Firmenich SA, Geneva, Switzerland), Romandolide^{®} ((15,1'R)-[1-(3',3'-Dimethyl-1'-cyclohexyl)ethoxycarbonyl]methyl propanoate, origin: Firmenich SA, Geneva, Switzerland), 2-tert-butyl-1-cyclohexyl acetate (origin: International Flavors and Fragrances, USA), Limbanol^{®} (1-(2,2,3,6-tetramethyl-cyclohexyl)-3-hexanol, origin: Firmenich SA, Geneva, Switzerland), trans-1-(2,2,6-trimethyl-1-cyclohexyl)-3-hexanol (origin: Firmenich SA, Geneva, Switzerland), (E)-3-methyl-4-(2,6,6-trimethyl-2-cyclohexen-1-yl)-3-buten-2-one, terpenyl isobutyrate, Lorysia^{®} (4-(1, 1-dimethylethyl)-1-cyclohexyl acetate, origin: Firmenich SA, Geneva, Switzerland), 8-methoxy-1-p-menthene, Helvetolide^{®} ((1S,1'R)-2-[1-(3',3'-dimethyl-1'-cyclohexyl) ethoxy]-2-methylpropyl propanoate, origin: Firmenich SA, Geneva, Switzerland), para tert-butylcyclohexanone, menthenethiol, 1-methyl-4-(4-methyl-3-pentenyl)-3-cyclohexene-1-carbaldehyde, allyl cyclohexylpropionate, cyclohexyl salicylate, 2-methoxy-4-methylphenyl methyl carbonate, ethyl 2-methoxy-4-methylphenyl carbonate, 4-ethyl-2-methoxyphenyl methyl carbonate;
- Group 4: Methyl cedryl ketone (origin: International Flavors and Fragrances, USA), Verdylate, vetyverol, vetyverone, 1-(octahydro-2,3,8,8-tetramethyl-2-naphtalenyl)-1-ethanone (origin: International Flavors and Fragrances, USA), (5RS,9RS,10SR)-2,6,9,10-tetramethyl-1-oxaspiro[4.5]deca-3,6-diene and the (5RS,9SR,10RS) isomer, 6-ethyl-2,10,10-trimethyl-1-oxaspiro[4.5]deca-3,6-diene, 1,2,3,5,6,7-hexahydro-1,1,2,3,3-pentamethyl-4-indenone (origin: International Flavors and Fragrances, USA), Hivemal^{®} (a mixture of 3-(3,3-dimethyl-5-indanyl)propanal and 3-(1,1-dimethyl-5-indanyl)propanal, origin: Firmenich SA, Geneva, Switzerland), Rhubofix^{®} (3',4-dimethyl-tricyclo[6.2.1.0(2,7)]undec-4-ene-9-spiro-2'-oxirane, origin: Firmenich SA, Geneva, Switzerland), 9/10-ethyldiene-3-oxatricyclo[6.2.1.0(2,7)]undecane, Polywood^{®} (perhydro-5,5,8A-trimethyl-2-naphthalenyl acetate, origin: Firmenich SA, Geneva, Switzerland), octalynol, Cetalox^{®} (dodecahydro-3a,6,6,9a-tetramethyl-naphtho[2,1-b]furan, origin: Firmenich SA, Geneva, Switzerland), tricyclo[5.2.1.0(2,6)]dec-3-en-8-yl acetate and tricyclo[5.2.1.0(2,6)]dec-4-en-8-yl acetate as well as tricyclo[5.2.1.0(2,6)]dec-3-en-8-yl propanoate and tricyclo[5.2.1.0(2,6)]dec-4-en-8-yl propanoate, (+)-(1S,2S,3S)-2,6,6-trimethyl-bicyclo[3.1.1]heptane-3-spiro-2'-cyclohexen-4'-one;

- Group 5: camphor, borneol, isobornyl acetate, 8-isopropyl-6-methyl-bicyclo[2.2.2]oct-5-ene-2-carbaldehyde, camphopinene, cedramber (8-methoxy-2,6,6,8-tetramethyl-tricyclo[5.3.1.0(1,5)]undecane, origin: Firmenich SA, Geneva, Switzerland), cedrene, cedrenol, cedrol, Florex^{®} (mixture of 9-ethylidene-3-oxatricyclo[6.2.1.0(2,7)]undecan-4-one and 10-ethylidene-3-oxatricyclo[6.2.1.0(2,7)]undecan-4-one, origin: Firmenich SA, Geneva, Switzerland), 3-methoxy-7,7-dimethyl-10-methylene-bicyclo[4.3.1]decane (origin: Firmenich SA, Geneva, Switzerland);
- Group 6: Cedroxyde^{®} (trimethyl-13-oxabicyclo-[10.1.0]-trideca-4,8-diene , origin: Firmenich SA, Geneva, Switzerland), Ambrettolide LG ((E)-9-hexadecen-16-olide, origin: Firmenich SA, Geneva, Switzerland), Habanolide^{®} (pentadecenolide, origin: Firmenich SA, Geneva, Switzerland), muscenone (3-methyl-(4/5)-cyclopentadecenone, origin: Firmenich SA, Geneva, Switzerland), muscone (origin: Firmenich SA, Geneva, Switzerland), Exaltolide^{®} (pentadecanolide, origin: Firmenich SA, Geneva, Switzerland), Exaltone^{®} (cyclopentadecanone, origin: Firmenich SA, Geneva, Switzerland), (1-ethoxyethoxy)cyclododecane (origin: Firmenich SA, Geneva, Switzerland), Astrotone, 4,8-cyclododecadien-1-one;
- Group 7: Lilial^{®} (origin: Givaudan SA, Vernier, Switzerland), rosinol.

According to an embodiment, the perfume comprises at least 30%, particularly at least 50%, more particularly at least 60% of ingredients selected from Groups 1 to 7, as defined above. According to an embodiment, said perfume comprises at least 30%, particularly at least 50% of ingredients from Groups 3 to 7, as defined above. According to an embodiment, said perfume comprises at least 30%, particularly at least 50% of ingredients from Groups 3, 4, 6 or 7, as defined above.

According to another embodiment, the perfume comprises at least 30%, particularly at least 50%, more particularly at least 60% of ingredients having a logP above 3, particularly above 3.5 and even more particularly above 3.75.

According to an embodiment, the perfume used in the invention contains less than 10% of its own weight of primary alcohols, less than 15% of its own weight of secondary alcohols and less than 20% of its own weight of tertiary alcohols. According to an embodiment, the perfume used in the invention does not contain any primary alcohols and contains less than 15% of secondary and tertiary alcohols.

The perfuming ingredients may be dissolved in a solvent of current use in the perfume industry. According to an embodiment, the solvent is not an alcohol. Examples of such solvents are diethyl phthalate, isopropyl myristate, Abalyn^{®} (rosin resins, available from Eastman), benzyl benzoate, ethyl citrate, limonene or other terpenes, or isoparaffins. According to an embodiment, the solvent is very hydrophobic and highly sterically hindered, like for example Abalyn^{®} or benzyl benzoate. According to an embodiment, the perfume comprises less than 30% of solvent. According to an embodiment, the perfume comprises less than 20% and even more particularly less than 10% of solvent, all these percentages being defined by weight relative to the total weight of the perfume. According to an embodiment, the perfume is essentially free of solvent.

According to an embodiment, the oil phase (or the oil-based core) comprises:
- 25-100wt% of a perfume oil comprising at least 15wt% of high impact perfume raw materials having a Log T<-4, and
- 0-75wt% of a density balancing material having a density greater than 1.07 g/cm³.

The nature of high impact perfume raw materials having a Log T<-4 and density balancing material having a density greater than 1.07 g/cm³ are described in WO2018115250, the content of which are included by reference.

According to a particular embodiment, the hydrophobic material is free of any active ingredient (such as perfume). According to this particular embodiment, it comprises, preferably consists of hydrophobic solvents, preferably chosen in the group consisting of isopropyl myristate, tryglycerides (e.g. Neobee^{®} MCT oil, vegetable oils), D-limonene, silicone oil, mineral oil, and mixtures thereof, with optionally hydrophilic solvents preferably chosen in the group consisting of 1,4 butanediol, benzyl alcohol, triethyl citrate, triacetin, benzyl acetate, ethyl acetate, propylene glycol (1,2-propanediol), 1,3-Propanediol, dipropylene glycol, glycerol, glycol ethers and mixtures thereof.

According to any one of the invention's embodiments, the hydrophobic material represents between about 10% and 60% w/w, or even between 15% and 45% w/w, by weight, relative to the total weight of the dispersion as obtained after step c).

According to a particular embodiment, the oil phase comprises an amino-acid derived polyisocyanate and a perfume or flavor oil.

The term "biocide" refers to a chemical substance capable of killing living organisms (e.g. microorganisms) or reducing or preventing their growth and/or accumulation. Biocides are commonly used in medicine, agriculture, forestry, and in industry where they prevent the fouling of, for example, water, agricultural products including seed, and oil pipelines. A biocide can be a pesticide, including a fungicide, herbicide, insecticide, algicide, molluscicide, miticide and rodenticide; and/or an antimicrobial such as a germicide, antibiotic, antibacterial, antiviral, antifungal, antiprotozoal and/or antiparasite.

As used herein, a "pest control agent" indicates a substance that serves to repel or attract pests, to decrease, inhibit or promote their growth, development or their activity. Pests refer to any living organism, whether animal, plant or fungus, which is invasive or troublesome to plants or animals, pests include insects notably arthropods, mites, spiders, fungi, weeds, bacteria and other microorganisms.

### Amino-acid derived polyisocyanate having at least two isocyanate functional groups

According to the invention, an amino-acid derived polyisocyanate having at least two isocyanate functional groups is dissolved in a hydrophobic material to form an oil phase.

The amino-acid derived polyisocyanate having at least two isocyanate functional groups is preferably an amino-acid- derived diisocyanate or an amino-acid- derived triisocyanate, preferably an amino-acid derived triisocyanate.

According to an embodiment, the amino-acid- derived diisocyanate is lysine diisocyanate. Lysine diisocyanate can be found from different suppliers such as Watson International Ltd or Alfa Aesar.

The amino-acid derived triisocyanate is preferably chosen in the group consisting of arginine triisocyanate, asparagine triisocyanate, proline triisocyanate, glutamine triisocyanate, lysine triisocyanate, lysine ethyl ester triisocyanate, lysine methyl ester triisocyanate, lysine propyl ester triisocyanate, or derivatives thereof, and mixtures thereof.

According to a particular embodiment, the amino-acid derived triisocyanate is a lysine triisocyanate (LTI), preferably L-Lysine triisocyanate.

Lysine triisocyanate has the following molecular formula C₁₁H₁₃N₃O₅ (2-Isocyanatoethyl 2,6-diisocyanatocaproate) and can be found from different suppliers such as Watson International Ltd Infine chemicals Co., Limited.

According to a particular embodiment, the amino-acid derived triisocyanate, preferably Lysine triisocyanate is purified.

This purification process (also called "discoloration" or "bleaching" process) removes the solid impurities in raw material and is well-known from the skilled person in the art. It can be carried out for example by mixing the amino acid derived triisocyanate with activated carbon followed by a filtration. Solvents such as MTBE (methyl tertiary-butyl ether) or ethyl acetate can be used during the purification process.

According to an embodiment, there is used an amount of between 0.5 and 20%, preferably between 5 and 10% of an amino-acid derived polyisocyanate, these percentages being defined by weight relative to the total weight of the oil phase.

### Additional polyisocyanate

According to a particular embodiment, the oil phase of step a) comprises at least a further non-amino-acid derived polyisocyanate having at least two isocyanate groups.

Said polyisocyanate may comprise up to 6, or even only 4 isocyanate functional groups. According to any of the above embodiments, said polyisocyanate contains at least three isocyanate functional groups.

Low volatility polyisocyanates are preferred because of their low toxicity.

The at least one polyisocyanate may be aliphatic, aromatic or a mixture of both aromatic and aliphatic polyisocyanates. In the case of mixtures of polyisocyanates, each member of the mixture has at least two isocyanate functional groups.

According to one embodiment, the at least one polyisocyanate is an aromatic polyisocyanate.

The term "aromatic polyisocyanate" is meant here as encompassing any polyisocyanate comprising an aromatic moiety. Preferably, it comprises a phenyl, a toluyl, a xylyl, a naphthyl or a diphenyl moiety, more preferably a toluyl or a xylyl moiety. Preferred aromatic polyisocyanates are biurets and polyisocyanurates, more preferably comprising one of the abovecited specific aromatic moieties. More preferably, the aromatic polyisocyanate is a polyisocyanurate of toluene diisocyanate (commercially available from Bayer under the tradename Desmodur^{®} RC), a trimethylol propane-adduct of toluene diisocyanate (commercially available from Bayer under the tradename Desmodur^{®} L75), a trimethylol propane-adduct of xylylene diisocyanate (commercially available from Mitsui Chemicals under the tradename Takenate^{®} D-110N). In a most preferred embodiment, the aromatic polyisocyanate is a trimethylol propane-adduct of xylylene diisocyanate.

According to another embodiment, said polyisocyanate is an aliphatic polyisocyanate.

The term "aliphatic polyisocyanate" is defined as a polyisocyanate which does not comprise any aromatic moiety. Preferred aliphatic polyisocyanates are a trimer of hexamethylene diisocyanate, a trimer of isophorone diisocyanate, a trimethylol propane-adduct of hexamethylene diisocyanate (available from Mitsui Chemicals) or a biuret of hexamethylene diisocyanate (commercially available from Bayer under the tradename Desmodur^{®} N 100), among which a biuret of hexamethylene diisocyanate is even more preferred.

According to another embodiment, said at least one polyisocyanate is in the form of a mixture of at least one aliphatic polyisocyanate and of at least one aromatic polyisocyanate, both comprising at least two or three isocyanate functional groups, such as a mixture of a biuret of hexamethylene diisocyanate with a trimethylol propane-adduct of xylylene diisocyanate, a mixture of a biuret of hexamethylene diisocyanate with a polyisocyanurate of toluene diisocyanate and a mixture of a biuret of hexamethylene diisocyanate with a trimethylol propane-adduct of toluene diisocyanate. Most preferably, it is a mixture of a biuret of hexamethylene diisocyanate with a trimethylol propane-adduct of xylylene diisocyanate.

In a preferred embodiment, the at least one aliphatic polyisocyanate and the at least one aromatic polyisocyanate are used in a respective molar ratio comprised between 80:20 and 10:90, preferably between 75:25 and 20:80, more preferably between 60:40 and 20:80, even more preferably between 60:40 and 30:70, most preferably between 45:55 and 30:70.

According to a particular embodiment, the oil phase comprises in addition to the amino-acid derived polyisocyanate, a polyisocyanate selected from the group consisting of a polyisocyanurate of toluene diisocyanate, a trimethylol propane-adduct of toluene diisocyanate and a trimethylol propane-adduct of xylylene diisocyanate, and mixtures thereof.

When a polyisocyanate is further added (in addition to the amino-acid derived polyisocyanate), it is added in an amount comprised between 0.1 and 20%, preferably between 0.5 and 2% by weight, based on the total weight of the oil phase.

In another step of the process, a dispersing phase comprising a stabilizer is prepared

### Dispersing phase

There is no restriction regarding the nature of the solvent that can be used in step b) as long as it can dissolve/disperse the stabilizer.

According to a particular embodiment, the dispersing phase comprises, preferably consists of water.

According to another particular embodiment, the content of water is below or equal to 10%, preferably below or equal to 5%, more preferably below or equal to 3% by weight based on the total weight of the dispersing phase.

According to a particular embodiment, the dispersing phase is free of water.

According to an embodiment, the dispersing phase comprises a solvent chosen in the group consisting of glycerol, 1,4-butanediol, ethylene glycol and mixtures thereof.

### Stabilizer

According to the invention, the stabilizer can be ionic or non-ionic.

Among the ionic stabilizer, one may cite gum Arabic, carboxymethyl cellulose, soy protein, sodium caseinate, gelatin, bovine serum albumin, sugar beet pectin, hydrolyzed soy protein, hydrolyzed sericin, Pseudocollagen, Biopolymer SA-N (INCI name : Hyaluronic Acid (and) Serum Albumen (and) Dextran Sulfate), Pentacare-NA PF (Hydrolyzed Wheat Gluten (and) Ceratonia Siliqua (Carob) Gum (and) Aqua (and) Sodium Dextran Sulfate (and) Bis-Hydroxyethyl Tromethamine (and) Phenoxyethanol (and) Ethylhexylglycerin), and mixtures thereof.

Among the non-ionic emulsifier, one may cite polyvinyl alcohol, modified polyvinyl alcohol, modified starch, modified cellulose, polysaccharides, and mixtures thereof.

According to a particular embodiment, the stabilizer is chosen in the group consisting of gum Arabic, modified starch, polyvinyl alcohol, polyvinylpyrolidone (PVP), carboxymethylcellulose (CMC), anionic polysaccharides, acrylamide copolymer, inorganic particles, protein such as soy protein, rice protein, whey protein, white egg albumin, sodium caseinate, gelatin, bovine serum albumin, hydrolyzed soy protein, hydrolyzed sericin, pseudocollagen, silk protein, sericin powder, and mixtures thereof.

According to any one of the above embodiments of the present invention, the dispersion comprises between about 0.1% and 5% w/w of at least a stabilizer, percentage being expressed on a w/w basis relative to the total weight of the dispersion as obtained after step c). In still another aspect of the invention, the dispersion comprises between about 0.1% and 2% w/w of at least a stabilizer. In still another aspect of the invention, the dispersion comprises between about 0.1% and 1% w/w of at least a stabilizer.

In another step of the process, a reactant can be added to the dispersion obtained in step c).

The reactant of step c) can be chosen in the group consisting of an amino acid, a polyamine, a polyol, and mixtures thereof.

According to an embodiment, microcapsules according to the present invention are polyurea-based capsules. According to this particular embodiment, interfacial polymerization is induced by addition of a polyamine.

Polyamine may be chosen in the group consisting of water soluble guanidine salts, guanidine, tris-(2-aminoethyl)amine, N,N,N',N'-tetrakis(3-aminopropyl)-1,4-butanediamine and N,N'-bis(3-aminopropyl)-ethylenediamine, polyaminoester and mixtures thereof.

According to a particular embodiment, the polyamine is a polyaminoester as defined previously. The polyaminoester is preferably chosen in the group consisting of:

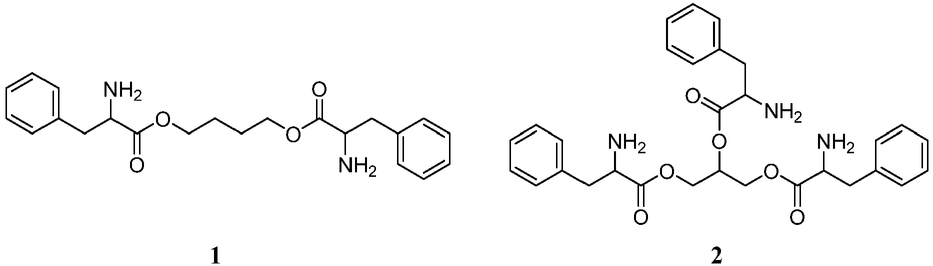

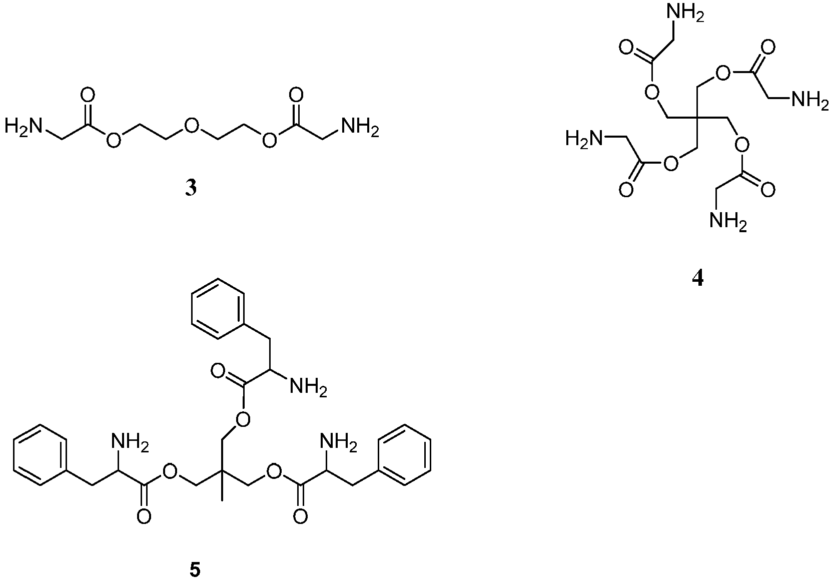

The polyamine is preferably used in an amount between 0.1 and 15%, more preferably between 4 and 12%, these percentages being defined by weight relative to the total weight of the slurry. According to a particular embodiment, the molar ratio between the amino groups of the polyamine and the isocyanate groups is comprised between 0.1 and 3, preferably between 0.5 and 1.

According to another embodiment, polyurea-based capsules are formed in absence of added polyamine reactant, and result only from the autopolymerization of the amino-acid derived polyisocyanate with the dispersing phase, preferably water.

According to another embodiment, the reactant is an amino acid comprising a functional group chosen in the group consisting of at least one NH₂ group, at least on OH group, at least one SH group and mixtures thereof.

According to an embodiment, the amino acid comprises at least two NH₂ groups, or one NH2 group and one OH group, or one NH₂ group and one SH group.

The amino acid is preferably chosen from the group consisting of lysine, arginine, cysteine, asparagine, glutamine, serine, threonine, cystine, and mixtures thereof.

According to another embodiment, microcapsules according to the present invention are polyurethane-based capsules. According to this particular embodiment, interfacial polymerization is induced by addition of a polyol reactant. Preferably the reactant is selected from the group consisting of monomeric and polymeric polyols with multiple hydroxyl groups available for reaction and mixtures thereof.

This is followed by a curing step e) which allows ending up with microcapsules in the form of a slurry.

According to a preferred embodiment, to enhance the kinetics, said step is performed at a temperature comprised between 60 and 80°C, possibly under pressure, for 1 to 4 hours. More preferably it is performed at between 50 and 90°C for between 30 minutes and 4 hours. However, the curing step can take place at room temperature.

### Optional outer coating

According to a particular embodiment of the invention, at the end of step e) or during step e), one may also add to the slurry a polymer selected from the group consisting of a nonionic polysaccharide, a cationic polymer and mixtures thereof to form an outer coating to the microcapsule.

Non-ionic polysaccharide polymers are well known to a person skilled in the art and are described for instance in WO2012/007438 page 29, lines 1 to 25 and in WO2013/026657 page 2, lines 12 to 19 and page 4, lines 3 to 12. Preferred non-ionic polysaccharides are selected from the group consisting of locust bean gum, xyloglucan, guar gum, hydroxypropyl guar, hydroxypropyl cellulose and hydroxypropyl methyl cellulose.

Cationic polymers are well known to a person skilled in the art. Preferred cationic polymers have cationic charge densities of at least 0.5 meq/g, more preferably at least about 1.5 meq/g, but also preferably less than about 7 meq/g, more preferably less than about 6.2 meq/g. The cationic charge density of the cationic polymers may be determined by the Kjeldahl method as described in the US Pharmacopoeia under chemical tests for Nitrogen determination. The preferred cationic polymers are chosen from those that contain units comprising primary, secondary, tertiary and/or quaternary amine groups that can either form part of the main polymer chain or can be borne by a side substituent directly connected thereto. The weight average (Mw) molecular weight of the cationic polymer is preferably between 10,000 and 3.5M Dalton, more preferably between 50,000 and 1.5M Dalton. According to a particular embodiment, one will use cationic polymers based on acrylamide, methacrylamide, N-vinylpyrrolidone, quaternized N,N-dimethylaminomethacrylate, diallyldimethylammonium chloride, quaternized vinylimidazole (3-methyl-1-vinyl-1H-imidazol-3-ium chloride), vinylpyrrolidone, acrylamidopropyltrimonium chloride, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride. Preferably copolymers shall be selected from the group consisting of polyquaternium-5, polyquaternium-6, polyquaternium-7, polyquaternium10, polyquaternium-11, polyquaternium-16, polyquaternium-22, polyquaternium-28, polyquaternium-43, polyquaternium-44, polyquaternium-46, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride. As specific examples of commercially available products, one may cite Salcare^{®} SC60 (cationic copolymer of acrylamidopropyltrimonium chloride and acrylamide, origin: BASF) or Luviquat^{®}, such as the PQ 11N, FC 550 or Style (polyquaternium-11 to 68 or quaternized copolymers of vinylpyrrolidone origin: BASF), or also the Jaguar^{®} (C13S or C17, origin Rhodia).

According to any one of the above embodiments of the invention, there is added an amount of polymer described above comprised between about 0% and 5% w/w, or even between about 0.1% and 2% w/w, percentage being expressed on a w/w basis relative to the total weight of the slurry as obtained after step d). It is clearly understood by a person skilled in the art that only part of said added polymers will be incorporated into/deposited on the microcapsule shell.

Another object of the invention is a process for preparing a microcapsule powder comprising the steps as defined above and an additional step f) consisting of submitting the slurry obtained in step e) to a drying, like spray-drying, to provide the microcapsules as such, i.e. in a powdery form. It is understood that any standard method known by a person skilled in the art to perform such drying is also applicable. In particular the slurry may be spray-dried preferably in the presence of a polymeric carrier material such as polyvinyl acetate, polyvinyl alcohol, dextrins, natural or modified starch, vegetable gums, pectins, xanthans, alginates, carragenans or cellulose derivatives to provide microcapsules in a powder form.

However, one may cite also other drying method such as the extrusion, plating, spray granulation, the fluidized bed, or even a drying at room temperature using materials (carrier, desiccant) that meet specific criteria as disclosed in WO2017/134179.

According to a particular embodiment, the carrier material contains free perfume oil which can be the same or different from the perfume from the core of the microcapsules.

Disclosed but not claimed is a microcapsule slurry obtainable by the process as described above.

### Microcapsule/ Microcapsule slurry

The composition of the shell enables to provide microcapsules that show the desired stability in the product base (e.g. counteracts efficiently the extraction of the hydrophobic material by the surfactants of the consumer product).

Thus, another object of the invention is a core-shell microcapsule comprising:
- an oil-based core comprising a hydrophobic material, preferably comprising a perfume oil, and
- a shell comprising at least a polymerized amino-acid derived polyisocyanate having at least two isocyanate functional groups: wherein the amino-acid derived polyisocyanate is lysine diisocyanate or chosen in the group consisting of arginine triisocyanate, asparagine triisocyanate, proline 5 triisocyanate, glutamine triisocyanate, lysine triisocyanate, lysine ethyl ester triisocyanate, lysine methyl ester triisocyanate, lysine propyl ester triisocyanate, or derivatives thereof, and mixtures thereof.

Disclosed but not claimed is a core-shell microcapsule slurry comprising at least one microcapsule made of:
- an oil-based core comprising a hydrophobic material, preferably comprising a perfume oil, and
- a shell comprising at least a polymerized amino-acid derived polyisocyanate having at least two isocyanate functional groups.

Another object of the invention is a solid particle comprising:
- a polymeric carrier material, preferably chosen in the group consisting of polyvinyl acetate, polyvinyl alcohol, dextrins, natural or modified starch, vegetable gums, pectins, xanthans, alginates, carragenans, cellulose derivatives and mixtures thereof, and
- microcapsules as defined above entrapped in said carrier material, and
- optionally free perfume entrapped in said carrier material.

Solid particle as defined above and microcapsule powder are used indifferently in the present invention.

The embodiments described previously for the process of preparing microcapsules also apply for the microcapsules defined above.

### Perfuming composition/consumer products

The microcapsules of the invention can be used in combination with active ingredients. An object of the invention is therefore a composition comprising:
(i) microcapsules as defined above;
(ii) an active ingredient, preferably chosen in the group consisting of a cosmetic ingredient, skin caring ingredient, perfume ingredient, flavor ingredient, malodour counteracting ingredient, pharmaceutical or agrochemical ingredient, a sanitizing ingredient, an insect repellent or attractant, and mixtures thereof.

The capsules of the invention show a good performance in terms of stability in challenging medium.

Another object of the present invention is a perfuming composition comprising:
(i) microcapsules as defined above, wherein the oil comprises a perfume;
(ii) at least one ingredient selected from the group consisting of a perfumery carrier, a perfumery co-ingredient and mixtures thereof;
(iii) optionally at least one perfumery adjuvant.

As liquid perfumery carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples solvents such as dipropyleneglycol, diethyl phthalate, isopropyl myristate, benzyl benzoate, 2-(2-ethoxyethoxy)-1-ethanol or ethyl citrate, which are the most commonly used. For the compositions which comprise both a perfumery carrier and a perfumery co-ingredient, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company). By "perfumery co-ingredient" it is meant here a compound, which is used in a perfuming preparation or a composition to impart a hedonic effect and which is not a microcapsule as defined above. In other words such a co-ingredient, to be considered as being a perfuming one, must be recognized by a person skilled in the art as being able to at least impart or modify in a positive or pleasant way the odor of a composition, and not just as having an odor.

The nature and type of the perfuming co-ingredients present in the perfuming composition do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

Preferably, the perfuming composition according to the invention comprises between 0.01 and 30 % by weight of microcapsules as defined above.

The invention's microcapsules can advantageously be used in many application fields and used in consumer products. Microcapsules can be used in liquid form applicable to liquid consumer products as well as in powder form, applicable to powder consumer products.

According to a particular embodiment, the consumer product as defined above is liquid and comprises:
a) from 2 to 65% by weight, relative to the total weight of the consumer product, of at least one surfactant;
b) water or a water-miscible hydrophilic organic solvent; and
c) microcapsule slurry or microcapsules as defined above,
d) optionally non-encapsulated perfume.

According to a particular embodiment, the consumer product as defined above is in a powder form and comprises:
a) from 2 to 65% by weight, relative to the total weight of the consumer product, of at least one surfactant;
b) microcapsules powder as defined above.
c) optionally perfume powder that is different from the microcapsules defined above.

In the case of microcapsules including a perfume oil-based core, the products of the invention, can in particular be of used in perfumed consumer products such as product belonging to fine fragrance or "functional" perfumery. Functional perfumery includes in particular personal-care products including hair-care, body cleansing, skin care, hygiene-care as well as home-care products including laundry care and air care. Consequently, another object of the present invention consists of a perfumed consumer product comprising as a perfuming ingredient, the microcapsules defined above or a perfuming composition as defined above. The perfume element of said consumer product can be a combination of perfume microcapsules as defined above and free or non-encapsulated perfume, as well as other types of perfume microcapsule than those here-disclosed.

In particular a liquid consumer product comprising:
a) from 2 to 65% by weight, relative to the total weight of the consumer product, of at least one surfactant;
b) water or a water-miscible hydrophilic organic solvent; and
c) a perfuming composition as defined above is another object of the invention.

Also a powder consumer product comprising
(a) from 2 to 65% by weight, relative to the total weight of the consumer product, of at least one surfactant; and
(b) a perfuming composition as defined above is part of the invention.

The invention's microcapsules can therefore be added as such or as part of an invention's perfuming composition in a perfumed consumer product.

For the sake of clarity, it has to be mentioned that, by "perfumed consumer product" it is meant a consumer product which is expected to deliver among different benefits a perfuming effect to the surface to which it is applied (e.g. skin, hair, textile, paper, or home surface) or in the air (air-freshener, deodorizer etc). In other words, a perfumed consumer product according to the invention is a manufactured product which comprises a functional formulation also referred to as "base", together with benefit agents, among which an effective amount of microcapsules according to the invention.

The nature and type of the other constituents of the perfumed consumer product do not warrant a more detailed description here, which in any case would not be exhaustive, the skilled person being able to select them on the basis of his general knowledge and according to the nature and the desired effect of said product. Base formulations of consumer products in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

Non-limiting examples of suitable perfumed consumer product can be a perfume, such as a fine perfume, a cologne, an after-shave lotion, a body-splash; a fabric care product, such as a liquid or solid detergent, tablets and pods, a fabric softener, a dryer sheet, a fabric refresher, an ironing water, or a bleach; a personal-care product, such as a hair-care product (e.g. a shampoo, hair conditioner, a colouring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product); an air care product, such as an air freshener or a "ready to use" powdered air freshener; or a home care product, such all-purpose cleaners, liquid or power or tablet dishwashing products, toilet cleaners or products for cleaning various surfaces, for example sprays & wipes intended for the treatment / refreshment of textiles or hard surfaces (floors, tiles, stone-floors etc.); a hygiene product such as sanitary napkins, diapers, toilet paper.

Another object of the invention is a consumer product comprising:
- a personal care active base, and
- microcapsules as defined above or the perfuming composition as defined above,
wherein the consumer product is in the form of a personal care composition.

Personal care active base in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

The personal care composition is preferably chosen in the group consisting of a hair-care product (e.g. a shampoo, hair conditioner, a colouring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product);

Another object of the invention is a consumer product comprising:
- a home care or a fabric care active base, and
- microcapsules as defined above or the perfuming composition as defined above,
wherein the consumer product is in the form of a home care or a fabric care composition.

Home care or fabric care active base in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

Preferably, the consumer product comprises from 0.1 to 15 wt%, more preferably between 0.2 and 5 wt% of the microcapsules of the present invention, these percentages being defined by weight relative to the total weight of the consumer product. Of course, the above concentrations may be adapted according to the benefit effect desired in each product.

According to a particular embodiment, the consumer product is in the form of a fabric softener composition and comprises:
- between 85 and 99.9% of a fabric softener active base;
- between 0.1 to 15 wt%, more preferably between 0.2 and 5 wt% by weight of the microcapsules of the invention.

The fabric softener active base may comprise cationic surfactants of quaternary ammonium, such as Diethyl ester dimethyl ammonium chloride (DEEDMAC), TEAQ (triethanolamine quat), HEQ (Hamburg esterquat) and mixtures thereof.

An object of the invention is a consumer product in the form of a fabric softener composition comprising:
- a fabric softener active base; preferably chosen in the group consisting of dialkyl quaternary ammonium salts, dialkyl ester quaternary ammonium salts (esterquats), Hamburg esterquat (HEQ), TEAQ (triethanolamine quat), silicones, cationic guars and mixtures thereof, preferably in an amount comprised between 85 and 99.95% by weight based on the total weight of the composition,
- microcapsules as defined above, preferably in an amount comprised between 0.05 to 15 wt%, more preferably between 0.1 and 5 wt% by weight based on the total weight of the composition.

An object of the invention is a consumer product in the form of a liquid detergent composition comprising:
- a liquid detergent active base; preferably chosen in the group consisting of anionic surfactant such as alkylbenzenesulfonate (ABS), secondary alkyl sulfonate (SAS), primary alcohol sulfate (PAS), lauryl ether sulfate (LES), methyl ester sulfonate (MES) and nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxydes, alkyl polyglucosides, alkyl polyglucosamides, preferably in an amount comprised between 85 and 99.95% by weight based on the total weight of the composition,
- the microcapsules as defined above, preferably in an amount comprised between 0.05 to 15 wt%, more preferably between 0.1 and 5 wt% by weight based on the total weight of the composition.

An object of the invention is a consumer product in the form of a solid detergent composition comprising:
- a solid detergent active base; preferably chosen in the group consisting of anionic surfactant such as alkylbenzenesulfonate (ABS), secondary alkyl sulfonate (SAS), primary alcohol sulfate (PAS), lauryl ether sulfate (LES), methyl ester sulfonate (MES) and nonionic surfactant such as alkyl amines, alkanolamide, fatty alcohol polyethylene glycol) ether, fatty alcohol ethoxylate (FAE), ethylene oxide (EO) and propylene oxide (PO) copolymers, amine oxydes, alkyl polyglucosides, alkyl polyglucosamides, preferably in an amount comprised between 85 and 99.95% by weight based on the total weight of the composition,
- the microcapsules as defined above, preferably in an amount comprised between 0.05 to 15 wt%, more preferably between 0.1 and 5 wt% by weight based on the total weight of the composition.

An object of the invention is a consumer product in the form of a solid scent booster comprising:
- a solid carrier, preferably chosen in the group consisting of urea, sodium chloride, sodium sulphate, sodium acetate, zeolite, sodium carbonate, sodium bicarbonate, clay, talc, calcium carbonate, magnesium sulfate, gypsum, calcium sulfate, magnesium oxide, zinc oxide, titanium dioxide, calcium chloride, potassium chloride, magnesium chloride, zinc chloride, saccharides such as sucrose, mono-, di-, and polysaccharides and derivatives such as starch, cellulose, methyl cellulose, ethyl cellulose, propyl cellulose, polyols/sugar alcohols such as sorbitol, maltitol, xylitol, erythritol, and isomalt, PEG, PVP, citric acid or any water soluble solid acid, fatty alcohols or fatty acids and mixtures thereof.
- the microcapsules as defined above, in a powdered form, preferably in an amount comprised between 0.05 to 15 wt%, more preferably between 0.1 and 5 wt% by weight based on the total weight of the composition.

An object of the invention is a consumer product in the form of a liquid scent booster comprising:
- an aqueous phase,
- a surfactant system essentially consisting of one or more than one non-ionic surfactant, wherein the surfactant system has a mean HLB between 10 and 14, preferably chosen in the group consisting of ethoxylated aliphatic alcohols, POE/PPG (polyoxyethylene and polyoxypropylene) ethers, mono and polyglyceryl esters, sucrose ester compounds, polyoxyethylene hydroxylesters, alkyl polyglucosides, amine oxides and combinations thereof;
- a linker chosen in the group consisting of alcohols, salts and esters of carboxylic acids, salts and esters of hydroxyl carboxylic acids, fatty acids, fatty acid salts, glycerol fatty acids, surfactant having an HLB less than 10 and mixtures thereof, and
- the microcapsules as defined above, preferably in an amount comprised between 0.05 to 15 wt%, more preferably between 0.1 and 5 wt% by weight based on the total weight of the composition.

An object of the invention is a consumer product in the form of a shampoo or a shower gel composition comprising:
- a shampoo or a shower gel active base; preferably chosen in the group consisting of sodium alkylether sulfate, ammonium alkylether sulfates, alkylamphoacetate, cocamidopropyl betaine, cocamide MEA, alkylglucosides and aminoacid based surfactants and mixtures thereof, preferably in an amount comprised between 85 and 99.95% by weight based on the total weight of the composition,
- the microcapsules as defined above, preferably in an amount comprised between 0.05 to 15 wt%, more preferably between 0.1 and 5 wt% by weight based on the total weight of the composition.

An object of the invention is a consumer product in the form of a rinse-off conditioner composition comprising:
- a rinse-off conditioner active base; preferably chosen in the group consisting of cetyltrimonium chloride, stearyl trimonium chloride, benzalkonium chloride, behentrimonium chloride and mixture thereof, preferably in an amount comprised between 85 and 99.95% by weight based on the total weight of the composition,
- the microcapsules as defined above, preferably in an amount comprised between 0.05 to 15 wt%, more preferably between 0.1 and 5 wt% by weight based on the total weight of the composition.

According to a particular embodiment, the consumer product is in the form of a perfuming composition comprising:
- 0.1 to 30%, preferably 0.1 to 20% of the microcapsules as defined above,
- 0 to 40%, preferably 3-40% of perfume, and
- 20-90, preferably 40-90% of ethanol, by weight based on the total weight of the perfuming composition.

The invention will now be further described by way of examples. It will be appreciated that the invention as claimed is not intended to be limited in any way by these examples.

### Examples

### Example 1

### Microcapsules preparation according to the invention

### Protocol

### General protocol of Lysine triisocyanate purification

Raw L-lysine triisocyanate (LTI - 20 g - Watson International LTD) is weighed into a flask covered with the alumina foil and combined with methyl tert-butyl ether (150 mL). Activated carbon (4 grams) is added and the mixture is stirred for 30 minutes. The mixture is filtered and the filtrate is isolated and concentrated under reduced pressure in a rotavap to give amber oil.

### General protocol for capsule synthesis:

The purified LTI (light brown oil, 3 g) (with optionally Takenate^{®} D110N) was dissolved in 30g of a perfume oil (perfume oil A for capsules A-D or perfume oil B for capsules E - see respectively Table A and Table B). The oil phase was then added to an aqueous solution (37g) comprising a stabilizer (1% gum arabic solution) and homogenized for 4 min using an Ultra-Turrax T25 disperser at 24000 rpm to form an O/W emulsion. The emulsion was pH adjusted to 10 using NaOH solution (counted as the aqueous phase). This emulsion was then stirred at 500 rpm using a mechanical overhead stirrer and an aqueous solution comprising optionally a reactant was slowly added over 1 hour. Once the addition was complete, the reaction temperature was gradually elevated to 70 °C over 1 h and was maintained at 70 °C for 2 h before being allowed to cool to room temperature.

### Perfume oil compositions

**Table 2: Perfume oil A composition**

| **Ingredient** | **Concentration (Weight %)** |
|---|---|
| methyl 2,2-dimethyl-6-methylene-1-cyclohexanecarboxylate ¹⁾ | 20 |
| ((+-)-3-(4-isopro(2Zpylphenyl)-2-methylpropanal ²⁾ | 20 |
| 2-tert-butyl-1-cyclohexyl acetate ³⁾ | 20 |
| 4-tert-butyl-1-cyclohexyl acetate ⁴⁾ | 20 |
| ((2Z)-2-phenyl-2-hexenenitrile ⁵⁾ | 20 |

| | |
|---|---|
| 1) Origin: Firmenich SA, Geneva, Switzerland; 2) Origin: Firmenich SA, Geneva, Switzerland; 3) Origin: International Flavors and Fragrances, USA; 4) Origin: International Flavors and Fragrances, USA; 5) Origin: Firmenich SA, Geneva, Switzerland | |

**Table B: Perfume oil B composition**

| **Ingredient** | **Concentration (Weight %)** |
|---|---|
| 2,4-Dimethyl-3-cyclohexene-1-carbaldehyde | 3.30% |
| Allyl Heptanoate | 5.50% |
| Allyl amyl glycolate | 10.99% |
| Delta Damascone | 1.65% |
| Verdyl acetate | 20.30% |
| Methyl dihydrojasmonate ¹⁾ | 4.95% |
| 1-(octahydro-2,3,8,8-tetramethyl-2-naphtalenyl)-1-ethanone ²⁾ | 16.49% |
| Ald. Hexylcinnamique | 9.89% |
| Ethyl-2-methylvalerate | 3.3% |
| Lilial | 21.98% |
| (3Z)-3-hexen-1-yl butyrate | 1.1% |
| (-)-(8R)-8,12-epoxy-13,14,15,16-tetranorlabdane ³⁾ | 0.55% |
| Total | 100% |

| | |
|---|---|
| 1) Firmenich SA, Geneva, Switzerland 2) International Flavors & Fragrances, USA 3) Firmenich SA, Geneva, Switzerland | |

### Microcapsule slurry compositions

**Table 1: Microcapsules A composition**

| **Ingredient** | **Amount/g** | **Weight%** |
|---|---|---|
| Perfume | 30.00 | 38.40% |
| L-Lysine triisocyanate | 3.00 | 3.84% |
| Lysine (98%) | 2.12 | 2.71% |
| 0.5M NaB₄O₇ pH 9 buffer (10.1%NaB₄O₇) | 6.00 | 7.68% |
| 1% Superstab AA Gum Acacia Solution | 37.00 | 47.36% |
| **Total** | 78.12 | 100.0% |

**Figure 1** (JEOL 6010LA scanning electron microscope (SEM)) shows that buckled microcapsules were nicely formed. Average particle size (determined with Mastersizer 3000 laser diffraction particle size analyser) is D [4,3] ~ 17 µm. Thermogravimetric Analysis from 30°C to 50°C (5°C/min) and hold at 50°C for 250 min shows that that the capsule retained 100% of perfume oil A after 250 min.

**Table 2: Microcapsules B composition**

| **Ingredient** | **Amount/g** | **Weight%** |
|---|---|---|
| Perfume | 30.00 | 37.65% |
| L-Lysine triisocyanate | 3.00 | 3.76% |
| Arginine (99%) | 1.69 | 2.12% |
| 0.5M NaB₄O₇ pH9 buffer (10.1%NaB₄O₇) | 8.00 | 10.04% |
| 1% Superstab AA Gum Acacia Solution | 37.00 | 46.43% |
| **Total** | 79.69 | 100.00% |

**Table 3: Microcapsules C composition**

| **Ingredient** | **Amount/g** | **Weight%** |
|---|---|---|
| Perfume | 30.00 | 39.96% |
| L-Lysine triisocyanate | 3.00 | 4.0% |
| Guanidine Carbonate | 1.50 | 2.0% |
| 1% Superstab AA Gum Acacia Solution | 37.00 | 49.28% |
| Water | 3.58 | 4.77% |
| **Total** | 75.08 | 100.0% |

**Table 4: Microcapsules D composition**

| **Ingredient** | **Amount/g** | **Weight%** |
|---|---|---|
| Perfume | 30.00 | 39.47% |
| L-Lysine triisocyanate | 3.00 | 3.95% |
| 0.5M NaB₄O₇ pH9 buffer (10.1%NaB₄O₇) | 6.00 | 7.89% |
| 1% Superstab AA Gum Acacia Solution | 37.00 | 48.68% |
| **Total** | 76.00 | 100.0% |

**Table 5: Microcapsules E composition**

| **Ingredient** | **Amount/g** | **Weight%** |
|---|---|---|
| Perfume | 30.00 | 36.17% |
| L-Lysine triisocyanate | 3.40 | 4.10% |
| Takenate D110N | 0.50 | 0.60% |
| Lysine (98%) | 2.84 | 3.42% |
| H₂O | 9.21 | 11.10% |
| 1% Superstab AA Gum Acacia Solution (pH4) | 37.00 | 44.61% |
| Total | 82.95 | 100.00% |

### Example 2

### Stability of microcapsules in a shower gel

Capsules slurry A (450mg) or capsule slurry E (450mg) has been introduced in a shower gel base (10g) having the composition described in Table 6 (for Capsule slurry A) or Table 7 (for Capsule slurry E) leading to 1.4% of perfume oil in the sample.

**Table 6: Shower gel base composition A**

| **Ingredients** | **Quantity** |
|---|---|
| Deionized water | 56.85 |
| EDETA B Powder | 0.05 |
| Glydant Plus Liquid | 0.2 |
| 1,2-Propylene Glycol | 2 |
| TEXAPON NSO IS | 27 |
| Citric Acid 20% aqueous solution | 1 |
| CREMOPHOR RH 40 | 1 |
| Perfume | 1 |
| Sodium Chloride | 0.7 |
| Sodium Benzoate | 0.25 |
| Sodium Salicylate | 0.25 |
| MERQUAT 550 | 0.2 |
| Glucamate LT | 1.5 |
| TEGO BETAIN F 50 | 8 |

**Table 7: Shower gel base composition B**

| **Ingredients** | **Quantity** |
|---|---|
| Deionized water | 49.35 |
| EDETA B Powder | 0.05 |
| CARBOPOL AQUA SF-1 POLYMER | 0.20 |
| Zetesol AO 328 U | 35.00 |
| SODIUM HYDROXIDE 20% aqueous solution | 1.00 |
| TEGO-BETAIN F 50 | 8.00 |
| KATHON CG | 0.10 |
| Citric Acid (40%) | 0.50 |

The amount of perfume having leaked out of the capsules was then measured by solvent extraction and GC-FID analysis (Table 8 and 9).

**Table 8: Leakage (%) of microcapsules A in a shower gel A (RT)**

| | Leakage (%) |
|---|---|
| Week 0 | 2.15% |
| Week 1 | 5.91% |
| Week 4 | 7.33% |

**Table 9: Leakage (%) of microcapsules E in a shower gel B (43°C)**

| | Leakage (%) |
|---|---|
| Week 0 | 1.49% |
| Week 2 | 15.22% |

Results displayed in Tables 8 and 9 show that the microcapsules of the invention show good stability in a surfactant-based product at RT and at 43°C.

## Claims

1. A process for the preparation of a core-shell microcapsule slurry comprising the following steps:
a) dissolving at least an amino-acid derived polyisocyanate having at least two isocyanate functional groups in a hydrophobic material, preferably a perfume to form an oil phase;
b) preparing a dispersing phase comprising a stabilizer, wherein the dispersing phase is not miscible with the oil phase;
c) adding the oil phase into the dispersing phase to form a two-phases dispersion;
d) optionally, adding a reactant to the dispersion obtained in step c); and
e) performing a curing step to form core-shell microcapsules in the form of a slurry.

2. The process according to claim 1, **characterized in that** a reactant chosen in the group consisting of an amino acid, a polyamine, a polyol, and mixtures thereof is added in step d).

3. The process according to claim 1 or 2, **characterized in that** the reactant is used an amount of between 0.1 and 15%, preferably between 4 and 12%, these percentages being defined by weight relative to the total weight of the slurry.

4. The process according to claim 2, **characterized in that** the reactant is a polyamine chosen in the group consisting of water soluble guanidine salts, guanidine, tris-(2-aminoethyl)amine, N,N,N',N'-tetrakis(3-aminopropyl)-1,4-butanediamine and N,N'-bis(3-aminopropyl)-ethylenediamine, a polyaminoester, and mixtures thereof.

5. The process according to claim 4, **characterized in that** the polyaminoester is chosen in the group consisting of

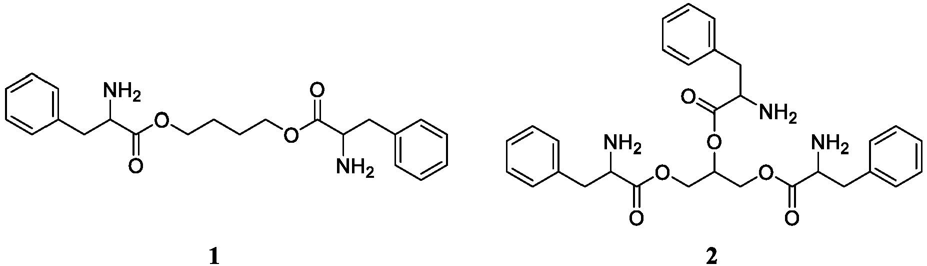

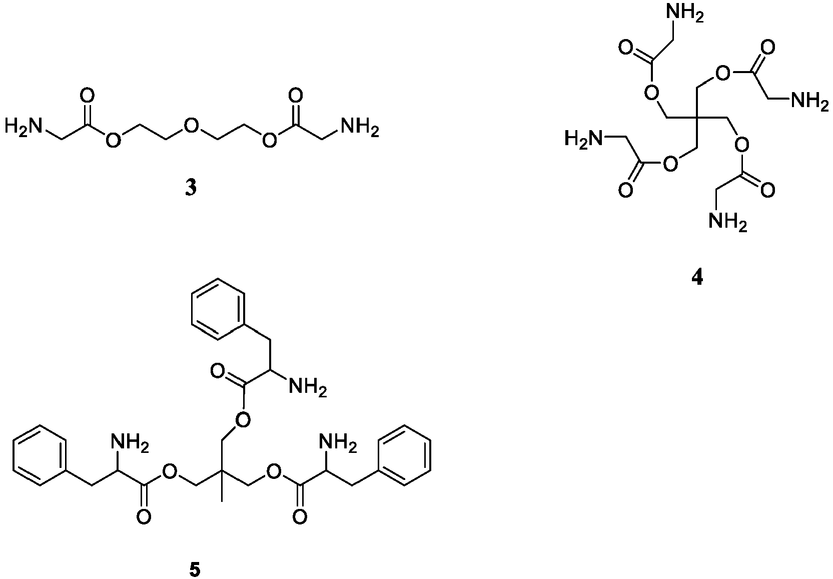

6. The process according to claim 2, **characterized in that** the reactant is an amino acid preferably chosen from the group consisting of lysine, arginine, cysteine, asparagine, glutamine, serine, threonine, cystine, and mixtures thereof.

7. The process according any one of the preceding claims, **characterized in that** the amino-acid derived polyisocyanate is chosen in the group consisting of an aminoacid derived triisocyanate, an amino-acid derived diisocyanate, and mixture thereof.

8. The process according to claim 7, **characterized in that** the amino-acid derived polyisocyanate is an amino-acid derived trisocyanate chosen in the group consisting of arginine triisocyanate, asparagine triisocyanate, proline triisocyanate, glutamine triisocyanate, lysine triisocyanate, lysine ethyl ester triisocyanate, lysine methyl ester triisocyanate, lysine propyl ester triisocyanate, or derivatives thereof, and mixtures thereof.

9. The process according to claim 8, **characterized in that** the amino-acid derived triisocyanate is lysine triisocyanate.

10. The process according to anyone of the preceding claims, **characterized in that** there is used an amount of between 0.5 and 20% of amino-acid derived polyisocyanate, these percentages being defined by weight relative to the total weight of the oil phase.

11. The process according to anyone of the preceding claims, **characterized in that** the oil phase of step a) further comprises at least one polyisocyanate selected from the group consisting of a polyisocyanurate of toluene diisocyanate, a trimethylol propane-adduct of toluene diisocyanate and a trimethylol propane-adduct of xylylene diisocyanate, and mixtures thereof.

12. The process according to anyone of the preceding claims, **characterized in that** the stabilizer is chosen in the group consisting of gum Arabic, modified starch, polyvinyl alcohol, polyvinylpyrolidone (PVP), carboxymethylcellulose (CMC), anionic polysaccharides, acrylamide copolymer, inorganic particles, protein such as soy protein, rice protein, whey protein, white egg albumin, sodium caseinate, gelatin, bovine serum albumin, hydrolyzed soy protein, hydrolyzed sericin, pseudocollagen, silk protein, sericin powder, and mixtures thereof.

13. A core-shell microcapsule comprising:
- an oil-based core comprising a hydrophobic material, preferably comprising a perfume oil, and
- a shell comprising at least a polymerized amino-acid derived polyisocyanate having at least two isocyanate functional groups;
wherein the amino-acid derived polyisocyanate is lysine diisocyanate or chosen in the group consisting of arginine triisocyanate, asparagine triisocyanate, proline triisocyanate, glutamine triisocyanate, lysine triisocyanate, lysine ethyl ester triisocyanate, lysine methyl ester triisocyanate, lysine propyl ester triisocyanate, or derivatives thereof, and mixtures thereof.

14. A perfuming composition comprising
(i) Perfume microcapsule as defined in claim 13, wherein the hydrophobic material comprises a perfume,
(ii) at least one ingredient selected from the group consisting of a perfumery carrier, a perfumery co-ingredient and mixtures thereof, and
(iii) Optionally at least one perfumery adjuvant.

15. A liquid perfumed consumer product comprising:
a) from 2 to 65% by weight, relative to the total weight of the consumer product, of at least one surfactant;
b) water or a water-miscible hydrophilic organic solvent; and
c) the microcapsule as defined in claim 13 or the perfuming composition as defined in claim 14.

## Patentansprüche

1. Verfahren zur Herstellung einer Kern-Hülle-Mikrokapsel-Aufschlämmung, das die folgenden Schritte umfasst:
a) Lösen von mindestens einem Aminosäure-abgeleiteten Polyisocyanat, das mindestens zwei funktionelle Isocyanatgruppen aufweist, in einem hydrophoben Material, vorzugsweise einem Parfüm, um eine Ölphase zu bilden;
b) Herstellen einer einen Stabilisator umfassenden dispergierenden Phase, wobei die dispergierende Phase nicht mit der Ölphase mischbar ist;
c) Zugeben der Ölphase in die dispergierende Phase, um eine zweiphasige Dispersion zu bilden;
d) wahlweises Zugeben eines Reaktanten zur in Schritt c) erhaltenen Dispersion; und
e) Durchführen eines Aushärtungsschritts, um Kern-Hülle-Mikrokapseln in der Form einer Aufschlämmung zu bilden.

2. Verfahren nach Anspruch 1, **dadurch gekennzeichnet, dass** ein Reaktant, ausgewählt aus der Gruppe, die aus einer Aminosäure, einem Polyamin, einem Polyol und Mischungen davon besteht, in Schritt d) zugegeben wird.

3. Verfahren nach Anspruch 1 oder 2, **dadurch gekennzeichnet, dass** der Reaktant in einer Menge zwischen 0,1 und 15 %, vorzugsweise zwischen 4 und 12 %, verwendet wird, wobei diese Prozentanteile nach Gewicht bezogen auf das Gesamtgewicht der Aufschlämmung definiert sind.

4. Verfahren nach Anspruch 2, **dadurch gekennzeichnet, dass** der Reaktant ein Polyamin ist, ausgewählt aus der Gruppe, die aus wasserlöslichen Guanidinsalzen, Guanidin, Tris-(2-aminoethyl)amin, N,N,N',N'-Tetrakis(3-aminopropyl)-1,4-butandiamin und N,N'-Bis(3-aminopropyl)-ethylendiamin, einem Polyaminoester und Mischungen davon besteht.

5. Verfahren nach Anspruch 4, **dadurch gekennzeichnet, dass** der Polyaminoester aus der Gruppe ausgewählt ist, die besteht aus

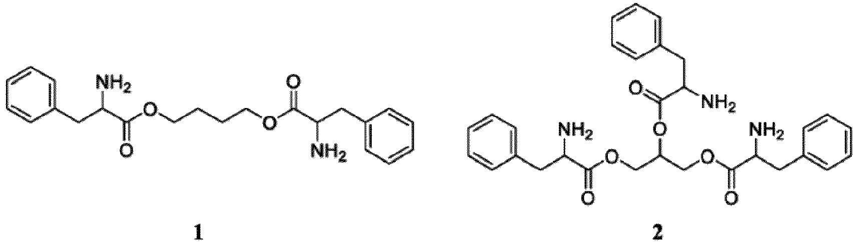

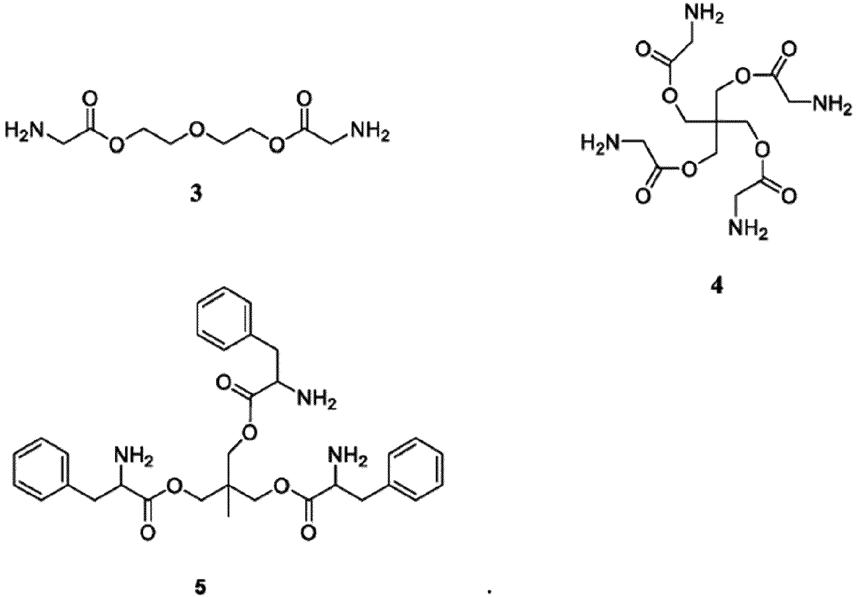

6. Verfahren nach Anspruch 2, **dadurch gekennzeichnet, dass** der Reaktant eine Aminosäure ist, vorzugsweise ausgewählt aus der Gruppe, die aus Lysin, Arginin, Cystein, Asparagin, Glutamin, Serin, Threonin, Cystin und Mischungen davon besteht.

7. Verfahren nach einem der vorstehenden Ansprüche, **dadurch gekennzeichnet, dass** das Aminosäure-abgeleitete Polyisocyanat aus der Gruppe ausgewählt ist, die aus einem Aminosäure-abgeleiteten Triisocyanat, einem Aminosäure-abgeleiteten Diisocyanat und Mischungen davon besteht.

8. Verfahren nach Anspruch 7, **dadurch gekennzeichnet, dass** das Aminosäure-abgeleitete Polyisocyanat ein Aminosäure-abgeleitetes Triisocyanat ist, ausgewählt aus der Gruppe, die besteht aus Arginintriisocyanat, Asparagintriisocyanat, Prolintriisocyanat, Glutamintriisocyanat, Lysintriisocyanat, Lysinethylestertriisocyanat, Lysinmethylestertriisocyanat, Lysinpropylestertriisocyanat, oder Derivaten davon, und Mischungen davon.

9. Verfahren nach Anspruch 8, **dadurch gekennzeichnet, dass** das Aminosäure-abgeleitete Triisocyanat Lysintriisocyanat ist.

10. Verfahren nach einem der vorstehenden Ansprüche, **dadurch gekennzeichnet, dass** eine Menge von zwischen 0,5 und 20 % Aminosäure-abgeleitetem Polyisocyanat verwendet wird, wobei diese Prozentanteile nach Gewicht bezogen auf das Gesamtgewicht der Ölphase definiert sind.

11. Verfahren nach einem der vorstehenden Ansprüche, **dadurch gekennzeichnet, dass** die Ölphase aus Schritt a) weiterhin mindestens ein Polyisocyanat umfasst, das aus der Gruppe ausgewählt ist, die besteht aus einem Polyisocyanurat von Toluoldiisocyanat, einem Trimethylolpropan-Addukt von Toluoldiisocyanat und einem Trimethylolpropan-Addukt von Xylylendiisocyanat sowie Mischungen davon.

12. Verfahren nach einem der vorstehenden Ansprüche, **dadurch gekennzeichnet, dass** der Stabilisator aus der Gruppe ausgewählt ist, die besteht aus Gummiarabikum, modifizierter Stärke, Polyvinylalkohol, Polyvinylpyrrolidon (PVP), Carboxymethylcellulose (CMC), anionischen Polysacchariden, Acrylamid-Copolymer, anorganischen Teilchen, Protein, wie z. B. Sojaprotein, Reisprotein, Molkenprotein, Eiweißalbumin, Natriumcaseinat, Gelatine, bovinem Serumalbumin, hydrolysiertem Sojaprotein, hydrolysiertem Sericin, Pseudocollagen, Seidenprotein, Sericinpulver und Mischungen davon.

13. Kern-Hülle-Mikrokapsel, umfassend:
- einen auf Öl basierenden Kern, umfassend ein hydrophobes Material, vorzugsweise umfassend ein Parfümöl, und
- eine Hülle, umfassend mindestens ein polymerisiertes Aminosäure-abgeleitetes Polyisocyanat, das mindestens zwei funktionelle Isocyanatgruppen aufweist;
wobei das Aminosäure-abgeleitete Polyisocyanat Lysindiisocyanat ist oder aus der Gruppe ausgewählt ist, die besteht aus Arginintriisocyanat, Asparagintriisocyanat, Prolintriisocyanat, Glutamintriisocyanat, Lysintriisocyanat, Lysinethylestertriisocyanat, Lysinmethylestertriisocyanat, Lysinpropylestertriisocyanat, oder Derivaten davon, und Mischungen davon.

14. Parfümierende Zusammensetzung, umfassend
(i) eine Parfümmikrokapsel gemäß Anspruch 13, wobei das hydrophobe Material ein Parfüm umfasst,
(ii) mindestens eine Ingredienz, ausgewählt aus der Gruppe, die aus einem Parfümträger, einer Parfüm-Koingredienz und Mischungen davon besteht, und
(iii) wahlweise mindestens einen Parfümhilfsstoff.

15. Flüssiges parfümiertes Verbraucherprodukt, umfassend:
a) 2 bis 65 Gew.- %, bezogen auf das Gesamtgewicht des Verbraucherprodukts, von mindestens einem Tensid;
b) Wasser oder ein wassermischbares hydrophiles organisches Lösungsmittel; und
c) die Mikrokapsel gemäß Anspruch 13 oder die parfümierende Zusammensetzung gemäß Anspruch 14.

## Revendications

1. Procédé pour la préparation d'une suspension de microcapsules type noyau-enveloppe comprenant les étapes suivantes :
a) dissolution d'au moins un polyisocyanate dérivé d'un acide aminé possédant au moins deux groupes fonctionnels isocyanates dans un matériau hydrophobe, de préférence un parfum, pour former une phase huileuse ;
b) préparation d'une phase de dispersion comprenant un stabilisant, dans lequel la phase de dispersion n'est pas miscible avec la phase huileuse ;
c) ajout de la phase huileuse dans la phase de dispersion pour former une dispersion biphasée ;
d) optionnellement, ajout d'un réactif à la dispersion obtenue dans l'étape c) ; et
e) réalisation d'une étape de durcissement pour former des microcapsules type noyau-enveloppe sous la forme d'une suspension.

2. Procédé selon la revendication 1, **caractérisé en ce qu'**un réactif choisi dans le groupe constitué de : un acide aminé, une polyamine, un polyol et des mélanges de ceux-ci est ajouté dans l'étape d).

3. Procédé selon la revendication 1 ou 2, **caractérisé en ce que** le réactif est utilisé en une quantité comprise entre 0,1 et 15 %, de préférence entre 4 et 12 %, ces pourcentages étant définis en poids relativement au poids total de la suspension.

4. Procédé selon la revendication 2, **caractérisé en ce que** le réactif est une polyamine choisie dans le groupe constitué de : sels de guanidine solubles dans l'eau, guanidine, tris-(2-aminoéthyl)amine, N,N,N',N'-tétrakis(3-aminopropyl)-1,4-butanediamine et N,N'-bis(3-aminopropyl)-éthylènediamine, un polyaminoester et des mélanges de ceux-ci.

5. Procédé selon la revendication 4, **caractérisé en ce que** le polyaminoester est choisi dans le groupe constitué de :

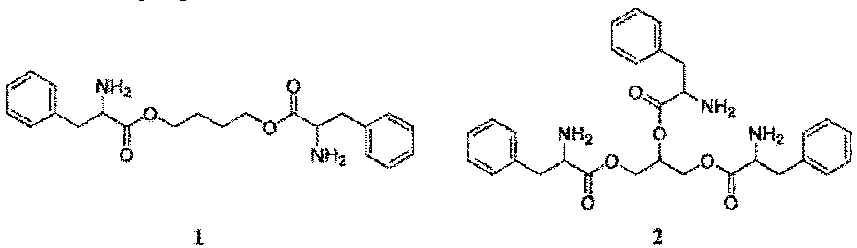

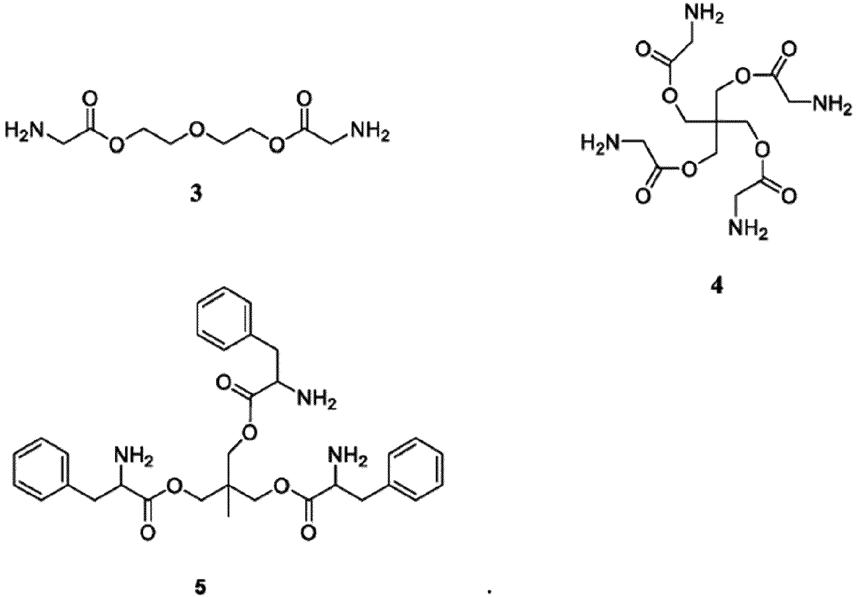

6. Procédé selon la revendication 2, **caractérisé en ce que** le réactif est un acide aminé choisi de préférence dans le groupe constitué de : lysine, arginine, cystéine, asparagine, glutamine, sérine, thréonine, cystine et des mélanges de ceux-ci.

7. Procédé selon l'une quelconque des revendications précédentes, **caractérisé en ce que** le polyisocyanate dérivé d'un acide aminé est choisi dans le groupe constitué de : un triisocyanate dérivé d'un acide aminé, un diisocyanate dérivé d'un acide aminé et des mélanges de ceux-ci.

8. Procédé selon la revendication 7, **caractérisé en ce que** le polyisocyanate dérivé d'un acide aminé est un triisocyanate dérivé d'un acide aminé choisi dans le groupe constitué de : triisocyanate d'arginine, triisocyanate d'asparagine, triisocyanate de proline, triisocyanate de glutamine, triisocyanate de lysine, triisocyanate de lysine éthylester, triisocyanate de lysine méthylester, triisocyanate de lysine propylester ou des dérivés de ceux-ci et des mélanges de ceux-ci.

9. Procédé selon la revendication 8, **caractérisé en ce que** le triisocyanate dérivé d'un acide aminé est le triisocyanate de lysine.

10. Procédé selon l'une quelconque des revendications précédentes, **caractérisé en ce qu'**il est utilisé une quantité comprise entre 0,5 et 20 % de polyisocyanate dérivé d'un acide aminé, ces pourcentages étant définis en poids relativement au poids total de la phase huileuse.

11. Procédé selon l'une quelconque des revendications précédentes, **caractérisé en ce que** la phase huileuse de l'étape a) comprend en outre au moins un polyisocyanate choisi dans le groupe constitué de : un polyisocyanurate de toluène diisocyanate, un adduit de triméthylol propane de toluène diisocyanate et un adduit de triméthylol propane de xylylène diisocyanate et des mélanges de ceux-ci.

12. Procédé selon l'une quelconque des revendications précédentes, **caractérisé en ce que** le stabilisant est choisi dans le groupe constitué de : gomme arabique, amidon modifié, alcool polyvinylique, polyvinylpyrolidone (PVP), carboxyméthylcellulose (CMC), polysaccharides anioniques, copolymère d'acrylamide, particules inorganiques, protéine telle que protéine de soja, protéine de riz, protéine de petit-lait, albumine de blanc d'oeuf, caséinate de sodium, gélatine, albumine de sérum bovin, protéine de soja hydrolysée, séricine hydrolysée, pseudo-collagène, protéine de soie, poudre de séricine et des mélanges de ceux-ci.

13. Microcapsule type noyau-enveloppe comprenant :
- un noyau à base d'huile comprenant un matériau hydrophobe, de préférence comprenant un parfum huileux, et
- une enveloppe comprenant au moins un polyisocyanate dérivé d'un acide aminé polymérisé possédant au moins deux groupes fonctionnels isocyanates ;
dans laquelle le polyisocyanate dérivé d'un acide aminé est le diisocyanate de lysine ou est choisi dans le groupe constitué de : triisocyanate d'arginine, triisocyanate d'asparagine, triisocyanate de proline, triisocyanate de glutamine, triisocyanate de lysine, triisocyanate de lysine éthylester, triisocyanate de lysine méthylester, triisocyanate de lysine propylester ou des dérivés de ceux-ci et des mélanges de ceux-ci.

14. Composition parfumante comprenant :
(i) une microcapsule de parfum selon la revendication 13, dans laquelle le matériau hydrophobe comprend un parfum,
(ii) au moins un ingrédient choisi dans le groupe constitué de : un support de parfumerie, un co-ingrédient de parfumerie et des mélanges de ceux-ci, et
(iii) optionnellement au moins un adjuvant de parfumerie.

15. Produit de consommation parfumé liquide comprenant :
a) 2 à 65 % en poids, relativement au poids total du produit de consommation, d'au moins un tensioactif ;
b) de l'eau ou un solvant organique hydrophile miscible avec l'eau ; et
c) la microcapsule selon la revendication 13 ou la composition parfumante selon la revendication 14.
